(19) Europäisches Patentamt
European Patent Office
Office européen des brevets

(11) EP 1 523 467 B1

(12) **EUROPÄISCHE PATENTSCHRIFT**

(45) Veröffentlichungstag und Bekanntmachung des Hinweises auf die Patenterteilung:
**14.11.2007 Patentblatt 2007/46**

(21) Anmeldenummer: **03763838.4**

(22) Anmeldetag: **14.07.2003**

(51) Int Cl.:
*C07C 45/62* (2006.01) *C07C 49/04* (2006.01)

(86) Internationale Anmeldenummer:
**PCT/EP2003/007600**

(87) Internationale Veröffentlichungsnummer:
**WO 2004/007413 (22.01.2004 Gazette 2004/04)**

(54) **VERFAHREN ZUR HERSTELLUNG VON TETRAHYDROGERANYLACETON**

METHOD FOR THE PRODUCTION OF TETRAHYDROGERANYLACETONE

PROCEDE DE PRODUCTION DE TETRAHYDROGERANYLACETONE

(84) Benannte Vertragsstaaten:
**AT BE BG CH CY CZ DE DK EE ES FI FR GB GR HU IE IT LI LU MC NL PT RO SE SI SK TR**

(30) Priorität: **15.07.2002 DE 10231945**

(43) Veröffentlichungstag der Anmeldung:
**20.04.2005 Patentblatt 2005/16**

(73) Patentinhaber: **BASF Aktiengesellschaft**
**67056 Ludwigshafen (DE)**

(72) Erfinder:
- **GÖBBEL, Hans-Georg**
  **67169 Kallstadt (DE)**
- **KAIBEL, Gerd**
  **68623 Lampertheim (DE)**
- **MILLER, Christian**
  **67152 Ruppertsberg (DE)**
- **DOBLER, Walter**
  **68723 Schwetzingen (DE)**
- **DIRNSTEINER, Thomas**
  **55116 Mainz (DE)**
- **HAHN, Thilo**
  **67071 Ludwigshafen (DE)**
- **BREUER, Klaus**
  **67122 Altrip (DE)**
- **AQUILA, Werner**
  **68309 Mannheim (DE)**

(74) Vertreter: **Reitstötter - Kinzebach**
**Patentanwälte**
**Sternwartstrasse 4**
**81679 München (DE)**

(56) Entgegenhaltungen:
**EP-A- 0 798 039**
**EP-A- 0 947 493**
**DD-A- 226 872**
**GB-A- 788 301**
**US-A- 2 272 122**

EP 1 523 467 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Tetrahydrogeranylaceton durch selektive Hydrierung von Pseudojonon, Geranylaceton und/oder Dihydrogeranylaceton.

Tetrahydrogeranylaceton (THGAC, Hexahydropseudojonon) wird als Ausgangsstoff für die Herstellung von Isophytol verwendet, welches seinerseits als Edukt für die Herstellung von Vitamin E und Vitamin K eingesetzt wird (s. z.B. Ullmann's Encyclopedia of Industrial Chemistry, 5th ed. on CD-Rom, "Vitamins", chapter 4.11).

Die GB 788,301 beschreibt ein Verfahren zur Herstellung von THGAC bei dem im letzten Schritt Geranylaceton oder Dihydrogeranylaceton (Tetrahydropseudojonon) zu THGAC hydriert wird.

Prinzipiell sollte THGAC auch durch Hydrierung von Pseudojonon zugänglich sein, das durch Umsetzung von Citral mit Aceton oder durch Umsetzung von Dihydrolinalool mit Diketen oder mit Isopropenylmethylether hergestellt werden kann (s. Ullmann's Encyclopedia of Industrial Chemistry, loc. cit.). Bei der Herstellung von THGAC aus Pseudojonon stellt sich jedoch das Problem, dass drei olefinische Doppelbindungen in Gegenwart einer Ketocarbonylgruppe selektiv hydriert werden müssen (siehe Schema 1).

Schema 1

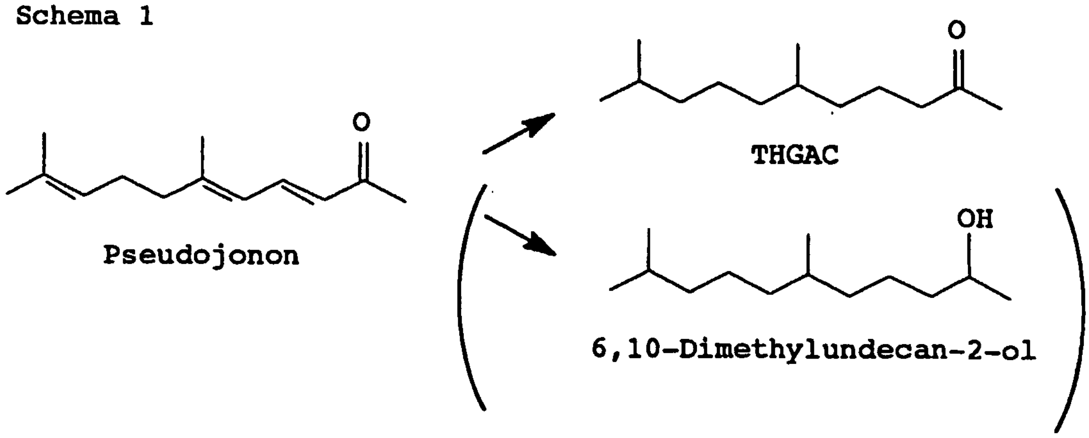

Pseudojonon

THGAC

6,10-Dimethylundecan-2-ol

Katalytische Hydrierungen an heterogenen Katalysatoren werden vielfach unter Einsatz von Festbettreaktoren durchgeführt, um die Vorzüge einer kontinuierlichen Verfahrensführung zu erhalten. Allerdings müssen dafür speziell präparierte Katalysatoren hergestellt und eingesetzt werden, die bei Verlust der Aktivität - oft bereits nach kurzeren Standzeiten - in aufwändiger Weise ausgetauscht oder regeneriert werden müssen, was in der Regel nicht nur mit der Abstellung der Hydrieranlage, sondern auch der nachfolgenden Aufarbeitungsstufen verbunden ist.

Alternativ kann eine heterogen katalysierte Hydrierung in Form einer Suspensionsreaktion durchgeführt werden, wobei der Hydrierkatalysator durch Zufuhr mechanischer Energie z.B. in einem Rührkessel in einer Flüssigphase suspendiert wird, vgl. z.B. Ullmanns Encyklopädie der technischen Chemie, 4. Aufl. Band 13, 1997, S. 138, Verlag Chemie Weinheim. Eine Erhöhung der Energiezufuhr über den zur Suspendierung erforderlichen Betrag führt zu keiner nennenswerten Verbesserung des Stoff transports der zu hydrierenden Moleküle an die Oberfläche der Katalysatorteilchen, da die erzielbare Relativgeschwindigkeit zwischen Katalysatorteilchen und Flüssigphase die Sedimentationsgeschwindigkeit nur unwesentlich übersteigt. Fließ- oder Wirbelbettreaktoren gestatten zwar höhere Relativgeschwindigkeiten, erfordern aber die Verwendung deutlich größerer Katalysatorteilchen, damit im Betrieb ein mehr oder weniger stark expandiertes Katalysatorbett vorliegt. Die geringere volumenbezogene Oberfläche größerer Katalysatorteilchen limitiert aber den Stoffumsatz und kompensiert so den Effekt der höheren Relativgeschwindigkeit.

Die US 5,939,589 beschreibt ein Verfahren zur Durchführung von katalytischen Reaktionen, bei dem eine flüssige Phase, in der ein Katalysator suspendiert vorliegt, und eine Gasphase im Reaktor durch eine Vorrichtung geführt werden, die Öffnungen oder Kanäle mit einem hydraulischen Durchmesser von 0,5 bis 20 mm aufweist. Beschrieben wird die Hydrierung von Hydrodehydrolinalool zu Hydrolinalool und weiter zu Tetrahydrolinalool. Hydrodehydrolinalool enthält lediglich eine Dreifachbindung als zu hydrierende funktionelle Gruppe, so dass der Fachmann dieser Schrift keine Anregung bezüglich einer selektiven Hydrierung entnommen hätte.

Der vorliegenden Erfindung liegt die Aufgabe zugrunde ein Verfahren zur Herstellung von THGAC aus Pseudojonon, Geranylaceton und/ oder Dihydrogeranylaceton bereitzustellen, das eine hohe Selektivität bezüglich der Hydrierung der C-C-Doppelbindungen aufweist, d.h. bei dem die Bildung des 6,10-Dimethylundecanols unter Reduktion der Carbonylgruppe möglichst unterdrückt ist, und das die Vorteile einer hohen Raum-Zeit-Ausbeute und eines einfachen Katalysatoraustausches vereint.

Die Aufgabe wird erfindungsgemäß gelöst durch ein Verfahren, bei dem man Pseudojonon, Geranylaceton und/oder Dihydrogeranylaceton, vorzugsweise Pseudojonon, in flüssiger Phase, in der Teilchen eines Katalysators suspendiert sind, der zur präferentiellen Hydrierung von Kohlenstoff-Kohlenstoff-Doppelbindungen vor Kohlenstoff-Sauerstoff-Doppelbindungen fähig ist, in Gegenwart eines wasserstoffhaltigen Gases durch eine Vorrichtung führt, welche den Transport der Katalysatorteilchen hemmt.

Beim erfindungsgemäßen Verfahren wird eine höhere Relativgeschwindigkeit der Flüssigphase gegenüber den Katalysatorteilchen erzeugt, weil der Transport der Katalysatorteilchen durch geeignete Mittel, wie Einbauten in einem Reaktor, gehemmt wird, d.h. die Partikel werden gegenüber der umgebenden Flüssigkeit stärker zurückgehalten. In Verbindung mit der hohen volumenbezogenen Oberfläche der suspendierten Partikel werden im Ergebnis hohe Raum-Zeit-Ausbeuten erzielt.

Eine geeignete Vorrichtung zur Durchführung des erfindungsgemäßen Verfahrens ist in der EP-A 798 039 beschrieben.

Die den Transport der Katalysatorteilchen hemmende Vorrichtung weist vorzugsweise Öffnungen oder Kanälen auf, deren hydraulischer Durchmesser das 2- bis 2000-fache, insbesondere das 5- bis 500-fache, besonders bevorzugt das 5- bis 100-fache des mittleren Durchmessers der Katalysatorteilchen beträgt.

Der hydraulische Durchmesser ist eine dem Fachmann geläufige Kenngröße zur Beschreibung des Äquivalentdurchmessers nichtkreisrunder Kanalstrukturen. Der hydraulische Durchmesser einer Öffnung ist als Quotient des 4-fachen Querschnitts der Öffnung und deren Umfang definiert. Bei Kanälen mit einem Querschnitt in Gestalt eines gleichschenkeligen Dreiecks lässt sich der hydraulische Durchmesser als

$$\frac{2bh}{b + 2s}$$

beschreiben, worin b für die Basis, h für die Höhe und s für die Schenkellänge des Dreiecks steht.

Die Öffnungen oder Kanäle geeigneter Vorrichtungen weisen im Allgemeinen einen hydraulischen Durchmesser von 0,5 bis 20 mm, vorzugsweise 1 bis 10 mm, besonders bevorzugt 1 bis 3 mm, auf.

Üblicherweise verwendet man Katalysatorteilchen eines mittleren Durchmessers von 0,0001 bis 2 mm, bevorzugt von 0,001 bis 1 mm, besonders bevorzugt von 0,005 bis 0,1 mm.

Die den Transport der Katalysatorteilchen hemmende Vorrichtung kann aus einer Schüttung, einem Gestrick, einer offenzelligen Schaumstruktur, vorzugsweise aus Kunststoff z.B. Polyurethan oder Melaminharz, oder Keramik, oder einem Packungselement, wie es grundsätzlich, d.h. seiner geometrischen Form nach, bereits aus der Destillations- und Extraktionstechnik bekannt ist, bestehen. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- und Extraktionstechnik.

Als Packungselemente eignen sich insbesondere Metallgewebepackungen bzw. Drahtgewebepackungen, z.B. der Bauart Montz A3, Sulzer BX, DX und EX. Anstelle von Metallgewebepackungen können auch Packungen aus anderen gewebten, gewirkten oder gefilzten Materialien verwendet werden. Weiterhin eignen sich Packungen ebener oder gewellter Bleche, bevorzugt ohne Perforation oder andere größere Öffnungen, beispielsweise entsprechend den Bauarten Montz B1 oder Sulzer Mellapak. Vorteilhaft sind auch Packungen aus Streckmetall, wie z.B. Packungen des Typs Montz BSH. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

In einer bevorzugten Ausführungsform weisen die der Flüssigphase zugewandten Oberflächen der Vorrichtung eine Rauhigkeit im Bereich des 0,1 bis 10-fachen, vorzugsweise des 0,5- bis 5-fachen, des mittleren Durchmessers der Katalysatorteilchen auf. Bevorzugt sind Materialien, deren Oberflächen einen Mittelrauwert $R_a$ (bestimmt nach DIN 4768/1) von 0,001 bis 0,01 mm aufweisen. Eine entsprechende Oberflächenrauhigkeit kann bei Verwendung von Drahtgewebepackungen aus Edelstahl durch thermische Behandlung in Gegenwart von Sauerstoff erreicht werden, z.B. indem man das Gewebe an der Luft bei einer Temperatur von etwa 800 °C tempert.

Die Flüssigphase enthält vorzugsweise wenigstens 80 Gew.-%, insbesondere wenigstens 90 Gew.-% Pseudojonon, d.h. sie enthält vorzugsweise keine größeren Mengen Verdünnungsmittel. Obgleich nicht bevorzugt, kann die Flüssigphase aber Verdünnungsmittel, wie z.B. $C_1$-$C_4$-Alkanole, z.B. Methanol enthalten.

Als wasserstoffhaltiges Gas verwendet man in der Regel Wasserstoffgas mit einer Reinheit von wenigstens 99,5 Vol.-%. Es wird in wenigstens stöchiometrischer Menge, bezogen auf die in der Flüssigphase enthaltene Carbonylverbindung eingesetzt, meist in einem Überschuß von 1 bis 20 %.

Als Katalysator kann ein handelsüblicher Suspensionskatalysator verwendet werden, der zur präferentiellen Hydrierung von Kohlenstoff-Kohlenstoff-Doppelbindungen vor Kohlenstoff-Sauerstoff-Doppelbindungen fähig ist. Es eignen sich besonders solche Katalysatoren, die als Aktivkomponente mindestens Palladium enthalten. Neben Palladium kann der Katalysator auch weitere Aktivkomponenten, wie beispielsweise Zink, Cadium, Platin, Silber oder ein Seltenerdmetall wie Cer, enthalten. Der Katalysator kann in metallischer und/oder oxidischer Form eingesetzt werden. Vorzugsweise sind die Aktivkomponenten auf einem Trägermaterial aufgebracht. Als Trägermaterialien eignen sich beispielsweise $SiO_2$ $TiO_2$, $ZrO_2$, $Al_2O_3$ oder Kohlenstoff wie Graphite, Ruße oder Aktivkohle. Aktivkohle ist aufgrund ihrer leichten Suspendierbarkeit bevorzugt. Der Gehalt an Palladium beträgt vorzugsweise 0,1 bis 10 Gew.-%, insbesondere 0,5 bis 7 Gew.-% und besonders bevorzugt 2 bis 6 Gew.-%, bezogen auf das Gesamtgewicht des Katalysators.

Das suspendierte Katalysatormaterial kann mit Hilfe gebräuchlicher Techniken in die Flüssigphase eingebracht und darin verteilt werden.

Bei der den Transport der Katalysatorteilchen hemmenden Vorrichtung handelt es sich üblicherweise um Einbauten in einem Reaktor, die so angeordnet sind, dass das Reaktionsgemisch beim Passieren des Reaktors durch die Vorrichtung gezwängt wird, d.h. die Einbauten füllen in der Regel den gesamten freien Querschnitt des Reaktors. Die Einbauten erstrecken sich vorzugsweise, aber nicht notwendigerweise über die gesamte Ausdehnung des Reaktors in Strömungsrichtung der Flüssigphase.

Es eignen sich verschiedene Reaktorformen, wie Strahldüsenreaktoren, Blasensäulen oder Rohrbündelreaktoren. Davon sind eine vertikal angeordnete Blasensäule oder ein Rohrbündelreaktor, bei dem die Einbauten in den einzelnen Rohren untergebracht sind, besonders geeignet.

Das wasserstoffhaltige Gas und die Flüssigphase werden bevorzugt im Gleichstrom, vorzugsweise entgegen der Richtung der Schwerkraft, durch den Reaktor geführt. Die Gasphase wird beispielsweise mittels einer Injektordüse innig mit der Flüssigphase durchmischt. Die Leerrohrgeschwindigkeit der Flüssigphase beträgt vorzugsweise mehr als 100 $m^3/m^2h$, insbesondere 100 bis 250 $m^3/m^2h$, die der Gasphase mehr als 100 $Nm^3/m^2h$, insbesondere 100 bis 250 $Nm^3/m^2h$. Um ausreichend hohe Leerrohrgeschwindigkeiten zu erzielen, ist es bevorzugt, Teilströme der Gas- und Flüssigphase, die den Reaktor verlassen, zurückzuführen.

Die im Hydrieraustrag suspendierten Katalysatorteilchen werden durch übliche Verfahren abgetrennt, z.B. durch Sedimentation, Zentrifugation, Kuchenfiltration oder Querstromfiltration.

Das Verfahren wird vorzugsweise bei einem Druck von 1 bis 100 bar, besonders bevorzugt von 1 bis 50 bar und insbesondere von 1 bis 20 bar durchgeführt. Die Reaktionstemperatur beträgt vorzugsweise 20 bis 150°C, besonders bevorzugt 20 bis 120°C und insbesondere 40 bis 80°C.

Das erfindungsgemäße Verfahren wird durch die beigefügte Figuren und das nachstehende Beispiel näher veranschaulicht.

Figur 1 zeigt schematisch eine zur Durchführung des erfindungsgemäßen Verfahrens geeignete Anlage mit einem Reaktor (Blasensäule) 1 mit einer Packung 2, die den Transport der Katalysatorteilchen hemmte. In den Reaktor werden über die Leitungen 3 Flüssigkeit und über die Leitung 4 Wasserstoffgas eingeführt. Das Kreisgas 5 wird mittels der Mischdüse 6 mit Frischgas und der durch die Pumpe 14 im Kreis geführten Suspension 11 eingemischt. Der Reaktoraustrag wird über die Leitung 7 in das Abscheidegefäß 8 gefahren, in dem die Gasphase abgeschieden und über Leitung 9 abgeführt wird. Von dieser Gasmenge wird zur Begrenzung der Aufpegelung von gasförmigen Verunreinigungen ein Teilstrom über die Leitung 10 entnommen und die verbleibende Restmenge über die Leitung 5 in den Reaktor geführt. Der suspendierte Katalysator verbleibt im Reaktorsystem, indem er über einen Querstromfilter 12 zurückgehalten und nur katalysatorfreie Flüssigphase über die Leitung 13 austritt und entnommen wird. Über den Wärmetauscher 15 kann die Temperatur im Reaktorsystem gezielt eingestellt werden.

Figur 2 zeigt schematisch eine Lage eines gefalzten Gewebes. Erfindungsgemäß verwendbare Packungen werden erhalten, wenn mehrere dieser Lagen übereinander angeordnet werden. Jede Lage umfasst Kanäle mit einem Querschnitt in Gestalt eines gleichschenkeligen Dreiecks mit der Schenkellänge s, der Basis b und der Höhe h.

Beispiele

Man verwendete eine Anlage wie in Fig. 1 dargestellt, die eine mit einer Gewebepackung vom Typ Montz A3 1200 bestückte Blasensäule (1000 mm Länge, 27,3 mm Durchmesser) umfasste. Die Packung bestand aus übereinander angeordneten Lagen eines Gewebe von Edelstahldrähten, das so gefalzt war, dass Kanäle mit einem Querschnitt in Gestalt eines gleichschenkeligen Dreiecks gebildet werden, wobei die Schenkellänge 3,1 mm, die Basis 5,1 mm und die Höhe 1,8 mm betrug, entsprechend einem hydraulischen Durchmesser von 1,62 mm.

Pseudojonon (97 gew.-%ig) mit dem darin suspendierten Katalysator und Wasserstoffgas wurden im Gleichstrom mit einer Leerrohrgeschwindigkeit von 200 $m^3/m^2*h$ von unten in den gepackten Reaktor eingebracht. Als Katalysator wurde ein handelsüblicher Pd-Kohle-Suspensionskatalysator mit einem Gehalt von 5 % Palladium auf Aktivkohle und einer mittleren Korngröße von 50 $\mu m$ verwendet.

Beispiel 1

Die Reaktion erfolgte kontinuierlich unter einem Wasserstoffdruck von 10 bar und bei einer Temperatur von 100°C. Man erhielt einen Umsatz von mehr als 99,9 %, wobei Tetrahydrogeranylaceton mit einer Selektivität von mehr als 96 %, bezogen auf die Gesamtproduktmenge, gebildet wurde. Die Selektivität zu 6,10-Dimethylundecan-2-ol betrug 3 %. Die Katalysatorbelastung betrug 2,5 $kg_{Pseudojonon}/(kg_{Katalysator}*h)$ ; die Raum-Zeit-Ausbeute betrug 500 $kg_{Pseudojonon}/(m^3*h)$.

Beispiel 2

Die Reaktion erfolgte kontinuierlich unter einem Wasserstoffdruck von 10 bar und bei einer Temperatur von 60°C. Man erhielt einen Umsatz von mehr als 99,9 %, wobei Tetrahydrogeranylaceton mit einer Selektivität von mehr als 96 %, bezogen auf die Gesamtproduktmenge, gebildet wurde. Die Selektivität zu 6,10-Dimethylundecan-2-ol betrug 0,3 %. Die Katalysatorbelastung betrug 2,5 $kg_{Pseudojonon}/(kg_{Katalysator}*h)$ ; die Raum-Zeit-Ausbeute betrug 500 $kg_{Pseudojonon}/(m^3*h)$.

## Patentansprüche

1. Verfahren zur Herstellung von Tetrahydrogeranylaceton, bei dem man eine Flüssigphase, die wenigstens 90 Gew.-% Pseudojonon umfasst und in der Teilchen eines Katalysators suspendiert sind, der zur präferentiellen Hydrierung von Kohlenstoff-Kohlenstoff-Doppelbindungen vor Kohlenstoff-Sauerstoff-Doppelbindungen fähig ist und dessen Aktivkomponente Palladium enthält, in Gegenwart eines wasserstoffhaltigen Gases durch eine Vorrichtung führt, welche den Transport der Katalysatorteilchen hemmt.

2. Verfahren nach Anspruch 1, wobei die den Transport der Katalysatorteilchen hemmende Vorrichtung Öffnungen oder Kanälen aufweist, deren hydraulischer Durchmesser das 2- bis 2000-fache des mittleren Durchmessers der Katalysatorteilchen beträgt.

3. Verfahren nach einem der vorhergehenden Ansprüche, wobei man Katalysatorteilchen eines mittleren Durchmessers von 0,0001 bis 2 mm verwendet.

4. Verfahren nach einem der vorhergehenden Ansprüche, wobei man als die den Transport der Katalysatorteilchen hemmende Vorrichtung eine Schüttung, ein Gestrick, eine offenzellige Schaumstruktur oder ein Packungselement verwendet.

5. Verfahren nach einem der vorhergehenden Ansprüche, wobei man die Flüssigphase und das wasserstoffhaltige Gas mit einer Leerrohrgeschwindigkeit von mehr als 100 $m^3/m^2h$ durch die den Transport der Katalysatorteilchen hemmende Vorrichtung führt.

6. Verfahren nach einem der vorhergehenden Ansprüche, wobei die der Flüssigphase zugewandten Oberflächen der Vorrichtung eine Rauhigkeit im Bereich des 0,1 bis 10-fachen des mittleren Durchmessers der Katalysatorteilchen aufweisen.

7. Verfahren nach einem der vorhergehenden Ansprüche, **dadurch gekennzeichnet, dass** der Reaktionsdruck 1 bis 100 bar beträgt.

8. Verfahren nach einem der vorhergehenden Ansprüche, **dadurch gekennzeichnet, dass** die Reaktionstemperatur 20 bis 120°C beträgt.

9. Verfahren nach Anspruch 5, **dadurch gekennzeichnet, dass** man Teilströme der Gas- und Flüssigphase, die den Reaktor verlassen, zurückführt.

## Claims

1. A process for preparing tetrahydrogeranylacetone in which a liquid phase which comprises at least 90% by weight of pseudoionone and in which particles of a catalyst which is capable of preferentially hydrogenating carbon-carbon

double bonds over carbon-oxygen double bonds and whose active component comprises palladium are suspended is conducted through a device which inhibits the transport of the catalyst particles in the presence of a hydrogen-containing gas.

**2.** The process according to claim 1, wherein the device inhibiting the transport of the catalyst particles has orifices or channels whose hydraulic diameter is from 2 to 2000 times the average diameter of the catalyst particles.

**3.** The process according to any of the preceding claims, wherein catalyst particles having an average diameter of from 0.0001 to 2 mm are used.

**4.** The process according to any of the preceding claims, wherein the device used for inhibiting the transport of the catalyst particles is a dumped packing, a knit, an open-celled foam structure or a structured packing element.

**5.** The process according to any of the preceding claims, wherein the liquid phase and the hydrogen-containing gas are conducted through the device which inhibits the transport of the catalyst particles at a superficial velocity of more than 100 $m^3/m^2h$.

**6.** The process according to any of the preceding claims, wherein the surfaces of the device facing toward the liquid phase have a roughness in the range from 0.1 to 10 times the average diameter of the catalyst particles.

**7.** The process according to any of the preceding claims, wherein the reaction pressure is from 1 to 100 bar.

**8.** The process according to any of the preceding claims, wherein the reaction temperature is from 20 to 120°C.

**9.** The process according to claim 5, wherein substreams of the gas and liquid phases which leave the reactor are recycled.

**Revendications**

**1.** Procédé de fabrication de tétrahydrogéranylacétone, par lequel une phase liquide comportant au moins 90 % en poids de pseudoionone et dans laquelle des particules d'un catalyseur sont suspendues, lequel est capable de l'hydrogénation préférentielle de liaisons doubles carbone-carbone avant des liaisons doubles carbone-oxygène et dont le composant actif comporte du palladium, est conduite, en présence d'un gaz hydrogéné, à travers un dispositif qui entrave le transport des particules de catalyseur.

**2.** Procédé selon la revendication 1, dans lequel le dispositif entravant le transport des particules de catalyseur présente des ouvertures ou des canaux dont le diamètre hydraulique est de 2 à 2000 fois le diamètre moyen des particules de catalyseur.

**3.** Procédé selon l'une quelconque des revendications précédentes, dans lequel sont utilisées des particules de catalyseur d'un diamètre moyen de 0,0001 à 2 mm.

**4.** Procédé selon l'une quelconque des revendications précédentes, dans lequel sont utilisés en tant que dispositif entravant le transport des particules de catalyseur un déversement, une structure tricotée, une structure de mousse à cellules ouvertes ou un élément de garnissage.

**5.** Procédé selon l'une quelconque des revendications précédentes, dans lequel la phase liquide et le gaz hydrogéné sont conduits à une vitesse de tube à vide supérieure à 100 $m^3/m^2h$ à travers le dispositif entravant le transport des particules de catalyseur.

**6.** Procédé selon l'une quelconque des revendications précédentes, dans lequel les surfaces du dispositif orientées vers la phase liquide présentent une rugosité de l'ordre de 0,1 à 10 fois le diamètre moyen des particules de catalyseur.

**7.** Procédé selon l'une quelconque des revendications précédentes, **caractérisé en ce que** la pression réactionnelle est de 1 à 100 bars.

**8.** Procédé selon l'une quelconque des revendications précédentes, **caractérisé en ce que** la température réactionnelle est de 20 à 120°C.

9. Procédé selon la revendication 5, **caractérisé en ce que** les écoulements partiels des phases gazeuses et liquides qui quittent le réacteur y sont réintroduits.

Fig. 1

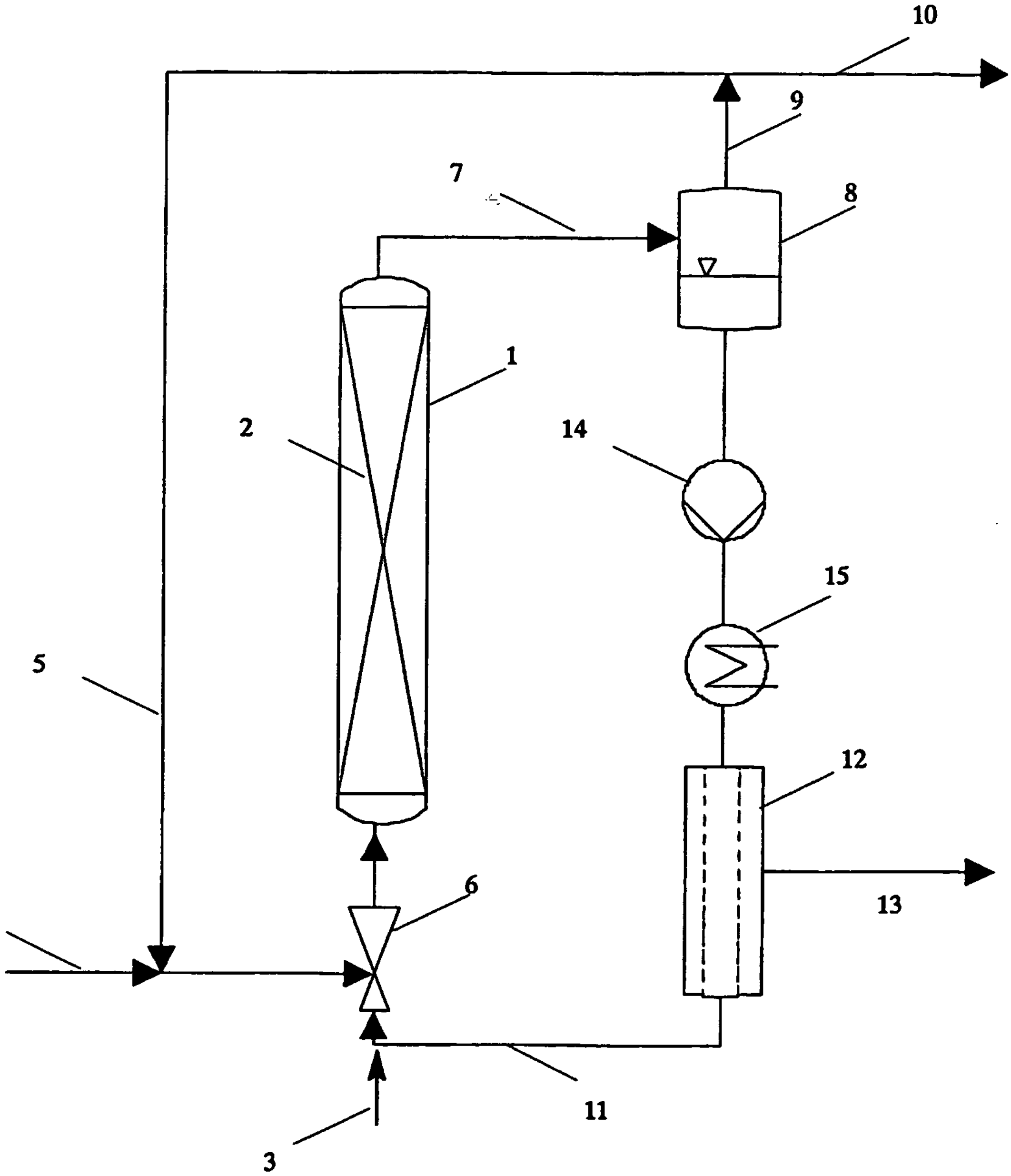

Fig. 2

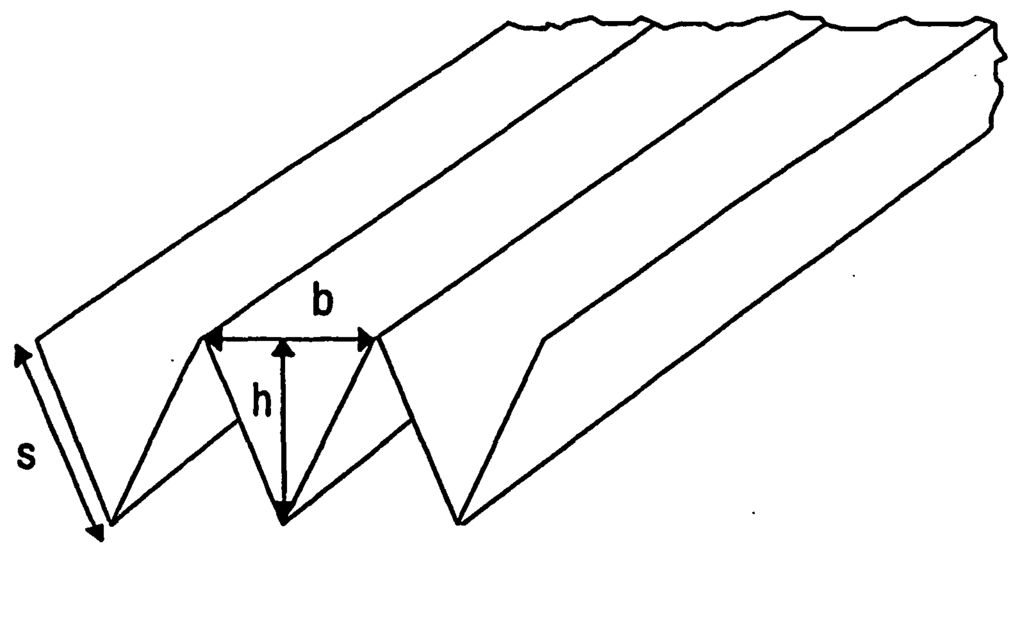

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 788301 A
- US 5939589 A
- EP 798039 A


### In der Beschreibung aufgeführte Nicht-Patentliteratur


- *Ullmanns Encyklopädie der technischen Chemie,* 1997, vol. 13, 138